# EUROPEAN PATENT APPLICATION

(11) **EP 1 442 738 A1**
(43) Date of publication of application: **04.08.2004**
(21) Application number: 03447019.5
(22) Date of filing: 28.01.2003
(51) Int. Cl.: A61K 7/48

(54) **Dermatological cosmetic composition comprising vitamin K1 oxide**

(71) Applicant: Auriga International S.A., 1410 Waterloo (BE)
(72) Inventor: Marchal, Alfred, 1410 Waterloo (BE)
(74) Representative: Van Malderen, Joelle

(57) **Abstract**

The present invention is related to a dermatological cosmetic composition comprising vitamin K1 oxide and its use for topical dermatological applications on skin.

## Description

### Field of the invention

The present invention is related to a dermatological cosmetic composition comprising vitamin K1 oxide and its use for topical dermatological applications on skin.

### Background of the invention and state of the art

Vitamin K1 (phylloquinone) is needed for proper bone formation and blood clotting, in both cases by helping the body transport calcium. Vitamin K (2-methyl 3-phytyl-1, 4-naphtoquinone) and its derivative has already been used in pharmaceutical or cosmetic compositions for its various anti-inflammatory or dermatological applications.

U.S. Patent No. 5,510,391 describes a method for treating blood vessel disorders of the skin using vitamin K. Such disorders include actinic and iatrogenic purpura, lentigines, telangiectasias of the face, spider angiomas and spider veins of the face.

However, the incorporation of vitamin K1 in a cosmetic composition is unstable in certain conditions when exposed to light and UV light and could modify the colour of cream and other vehicles of cosmetic compositions.

### Aims of the invention

A first aim of the present invention is to propose a new dermatological cosmetic composition with vitamin K1 derivative, which is more stable and which does not present the drawbacks of the state of the art.

A further aim of the present invention is to propose such composition which does not present the yellow colour of cream and vehicles already used in the state of the art and which therefore do not render the clothes of the consumer dirty.

A further aim of the present invention is to propose such a cosmetic composition, which is not sensitive to light or UV-radiation and which therefore decreases or eliminates consumer skin sensitivity or allergy following sun exposure.

A last aim of the present invention is to provide such a cosmetic composition with a similar or an improved activity (including enhancement penetration through the skin, excellent moisture-binding capacity,...) for dermatological applications than the cosmetic compositions of the state of the art.

### Sunanary of the invention

The present invention is related to a cosmetic composition (as described in the following claims and examples) and comprising a sufficient amount of vitamin K1 of following formula:

C₃₁H₄₆O₂ Mr 450.7 [84-80-0]

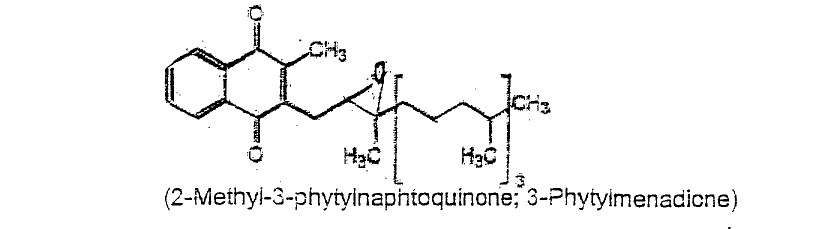

Said vitamin K1 is preferably a nano-sized vitamin K1 (about 180 nanometers in diameter).

### Examples

### Example 1: Protocol for bruises

For the purpose of the study, three cosmetic compositions have been used with different concentrations in vitamin K1 and in various vehicles and vitamin K1 oxide. The study was conducted in double blind on 12 volunteers people.

The study was performed with 12 people. Six males and six females. The purpose of the study was to show the best or the similar activity of vitamin K1 oxide versus vitamin K1 in resolution of bruises.

The 12 voluntaries previously received information on the goal and the course of the trial. Four ecchymosis have been induced on each patient (2 on each forearms) and the efficacy of each cream has been evaluated by observation of time reduction of each ecchymosis. Each patient was examined every other day and pictures have been taken at the same time. No other cream was applied and the patients were not allowed to take any other medication (no aspirin, no anti-inflammatory). The creams were applied twice a day.

### Materials and method

The table 1 presents 4 different creams and their composition.

**Table 1**

| **Creams** | **Composition** |
|---|---|
| cream 1 | 5% free vitamin K1 cream |
| cream 2 | 2% vitamin K gel under nanosome |
| cream 3 | 5% vitamin K oxide free cream |
| cream 4 | No treatment as witness |

Quantitative results in days of reduction of bruises are presented in the following table 2:

**Table 2**

| **Patient** | **Vitamin K1 in Nanosome 2% #2** | **Vitamin K1 of 5 % free #1** | **Vitamin K1 Oxyde % 5 % libre #3** | **No treatment #4** |
|---|---|---|---|---|
| A1 | 9 | 11 | 11 | 10 |
| A2 | 8 | 11 | 10 | 11 |
| A3 | 10 | 13 | 10 | 13 |
| A4 | 11 | 10 | 13 | 13 |
| A5 | 11 | 11 | 9 | 14 |
| A6 | 11 | 13 | 11 | 13 |
| B1 | 9 | 12 | 10 | 12 |
| B2 | 11 | 12 | 11 | 13 |
| B3 | 11 | 13 | 10 | 12 |
| B4 | 10 | 12 | 10 | 13 |
| B5 | 12 | 12 | 10 | 13 |
| B6 | 11 | 12 | 9 | 11 |
| | | | | |
| Total | 124 | 142 | 124 | 148 |
| **Mean** | **10,33** | **11,83** | **10,33** | **12,33** |

### Example 2: Protocol for spider veins

The same study was performed on 10 patients with spider veins or small broken blood vessels on legs and face.

The treatment was done during four weeks with two applications or the creams per day.

The group vitamin Oxide show the best results against other form of vitamin K1.

The trial show a very good tolerance of the various cream used.

The best formulation (which may include additional efficient cosmetic compounds (vitamin A, vitamin C, retinol, Propylene Glycol, Panthenol, Triethanolamine, Phytonadione, Lecithin, Carbomer, Ethoxydiglycol, Phospholipids, Ascorbic Palmitate, Retinyl palmitate, EDTA, Tocopherol, Acetate of tocopheryl, C12-C15 Alkyl benzoate, Caprylic capric triglycerides Parafimum liquidum, Cyclomethicone, Glycerine, Lecithin, Sodium PCA, Mica, Barium sulfate, Phytonadione, Titanium dioxide, Polysorbate 20, Acrylate copolymer, Phenoxyethanol, Acrylate C10-C30 alkyl crosspolymer, Propyl paraben, Menthyl paraben, BHT, BHA, ...)) can be easily identified in term of efficiency in the trial by the person skilled in the art as he performed in double blind Oxide vitamin K1 testing.

The cosmetic composition according to the invention is extremely suitable for the following dermatological application (upon eyes, legs, arms, ...) while presenting a similar or a better efficiency than the products of the state of the art.
- Bruises of any origins or associated with cosmetic surgery
- Vascular disorder on the skin such as small broken vessels
- Spider veins
- Varicoses
- Blotches on the face
- Any purpura on the face, body and legs such as actinic purpura, post laser skin treatment purpura
- Irritation following use of chemical peel
- Topical anti-inflammatory effect
- Shambourg's disease

Furthermore, the composition according to the invention presents the following advantageous characteristics:
- absence of yellow colour of creams or vehicles, which does not render the clothes of the consumer dirty,
- no sensibility of the cosmetic product or topical dermatological drugs regarding light or UV-radiation. Said last characteristic reduces or eliminates the patient's skin sensitivity or allergy following sun exposure.
- The composition according to the invention presents better or similar activities as phytonadione.
- The composition according to the invention is characterised by a high stability (especially topical creams, gels or solutions for topical application).

## Claims

1. An use of vitamin K1 oxide for topical dermatological applications.

2. The use of claim 1, wherein the vitamin K1 oxide is a nano-sized vitamin K1 (about 180 nanometers in diameter).

3. A cosmetic composition comprising a sufficient amount of vitamin K1 oxide and an adequate cosmetic carrier.

4. The cosmetic composition according to the claim 3, wherein said sufficient amount of vitamin K1 oxide is comprised between 0,5 and about 10%, preferably about 5% free vitamin K1 oxide.

5. The cosmetic composition of claim 3 or 4, wherein the vitamin K1 oxide is a nano-sized vitamin K1 (about 180 nanometers in diameter).

6. The cosmetic composition of claim 3, 4 or 5, wherein the cosmetic carrier is a cream, a gel, a lotion and/or a liquid.

7. An use of the cosmetic composition according to any of the claims 3 or 6 for topical dermatological applications upon a mammal skin, including a human skin.

8. The use according to the claim 7, wherein said dermatological application is selected from the group consisting of:
- bruises of any origins or associated with cosmetic surgery,
- vascular disorder on the skin such as small broken vessels,
- spider veins,
- varicoses,
- blotches on the face,
- any purpura on the face, body and legs such as actinic purpura, post laser skin treatment purpura,
- irritation following use of chemical peel,
- topical anti-inflammatory effect,
- Shambourg's disease or a mixture thereof
